**Europäisches Patentamt**

**European Patent Office**

**Office européen des brevets**

(11) Veröffentlichungsnummer: **0 225 571**
**A2**

## (12) EUROPÄISCHE PATENTANMELDUNG

(21) Anmeldenummer: **86116632.0**

(22) Anmeldetag: **29.11.86**

(51) Int. Cl.⁴: **F 25 J 1/02, H 01 K 3/22, B 01 J 19/14, B 65 D 90/44, H 01 J 9/395**

(30) Priorität: **07.12.85 DE 3543390**

(43) Veröffentlichungstag der Anmeldung: **16.06.87**
**Patentblatt 87/25**

(84) Benannte Vertragsstaaten: **AT BE CH DE ES FR GB GR IT LI NL SE**

(71) Anmelder: **Blaudszun, Bernd, Dipl.-Ing., Bürgerei 57 a, D-2162 Steinkirchen (DE)**
Anmelder: **Dittmar, Bernd, Im Wörthen 2, D-3105 Fassberg/Müden (DE)**

(72) Erfinder: **Blaudszun, Bernd, Dipl.-Ing., Bürgerei 57 a, D-2162 Steinkirchen (DE)**
Erfinder: **Dittmar, Bernd, Im Wörthen 2, D-3105 Fassberg/Müden (DE)**

(74) Vertreter: **Schmidt-Bogatzky, Jürgen, Dr. Ing., Schlossmühlendamm 4, D-2100 Hamburg 90 (DE)**

(54) **Verfahren zum Verflüssigen oder Verfestigen von bei Normaltemperatur in der Gasphase befindlichen kondensierbaren Stoffen insbesondere Edelgasen und Kohlenwasserstoffgasgemischen im Tieftemperaturbereich mittels eines Kühlmediums.**

(57) Die Erfindung betrifft ein Verfahren zur Verflüssigung oder Verfestigung von bei Normaltemperatur in der Gasphase befindlichen kondensierbaren Stoffen insbesondere Edelgasen und Kohlenwasserstoffgasgemischen im Tieftemperaturbereich mittels eines Kühlmediums, das den kondensierbaren Stoff in einem Wärmetauscher verflüssigt, eine Vorrichtung zur Durchführung des Verfahrens sowie die Anwendung des Verfahrens und der Vorrichtung. Verflüssigte Stoffe werden in einen Produkthohlkörper eingebracht, wobei das aus dem Wärmetauscher austretende, durch den Wärmeaustausch in der Gasphase befindliche Kühlmedium ganz oder teilweise auf die äußere Oberfläche des Produkthohlkörpers geleitet wird, wodurch der in diesem befindliche Stoff in der flüssigen Phase gehalten wird.

Die Erfindung betrifft ein Verfahren zum Verflüssigen oder Verfestigen von bei Normaltemperatur in der Gasphase befindlichen kondensierbaren Stoffen insbesondere Edelgasen und Kohlenwasserstoffgasgemischen im Tieftemperaturbereich mittels eines Kühlmediums, das den kondensierbaren Stoff in einem Wärmetauscher verflüssigt, die Anwendung des Verfahrens und eine Vorrichtung zur Durchführung des Verfahrens.

Es ist bekannt und üblich, zur Verflüssigung bzw. Verfestigung von Edelgasen oder Kohlenwasserstoffgasgemischen indirekte Verfahren zur Kühlung einzusetzen. Hierbei besteht der Nachteil, daß ein erheblicher Bedarf an Kühlmedium z. B. tiefkalt verflüssigten Inertgasen besteht, und hohe Betriebskosten anfallen. Zur Herstellung von z. B. Halogenlampen ist es erforderlich, daß die Halogenlampen nach dem Produktionsende einen relativ hohen Innendruck von ca. 4 bis 6 bar aufweisen. Die Füllung der Lampen besteht aus Edelgasen und speziellen Gasgemischen. Der Endfülldruck kann nur durch vorheriges Verflüssigen der Edelgase und Gasgemische erreicht werden. Üblicherweise werden zur Herstellung der Halogenlampen folgende Verfahrensschritte durchgeführt:

a. Reinigung der Lampenrohlinge durch Evakuieren,

b. Spülen der Lampenrohlinge durch reine Gase, vorzugsweise duch $N_2$,

c. Evakuieren der Lampenrohlinge,

d. Füllung der Lampenrohlinge mit speziellen Edelgasen oder Gasgemischen, wobei häufig Krypton verwendet wird,

e. Verflüssigung der Gasgemische durch Besprühen oder Tauchen der Lampenkörper mittels tiefkaltverflüssigter Inertgase, vorzugsweise verflüssigtem Stickstoff,

f. Kondensieren der Edelgase oder Gasgemische in den Lampenrohlingen,

g. Kalthalten der Lampenrohlinge zur Verhinderung der Rückvergasung kondensierter Edelgase oder Gasgemische, vorzugsweise durch Besprühen der Lampenrohlinge,

h. Verschweißen der Lampenrohlinge bei gleichzeitiger Aufrechterhaltung der Kühlung.

Die Verfahrensschritte b und c müssen unter Umständen mehrmals wiederholt werden. Für die Verfahrensschritte e bis h müssen tiefkalt verflüssigte Gase zur Kühlung eingesetzt werden. Vorzugsweise findet Flüssigstickstoff Anwendung. Diese Art der Kühlung ist sehr unwirtschaftlich, da 40% bis 50% des eingesetzten Kühlmediums nicht genutzt wird. Ursächlich hierfür ist die vorzeitige Verdampfung und/oder eine zuviel gesprühte Menge an Kühlmedium. Dieses hat hohe Betriebskosten und eine schlechte Reproduzierbarkeit der Verfahrensschritte zur Folge. Bisherige Versuche bei der Halogenlampenherstellung die Menge eingesetzter Kühlmedien einzusparen waren nicht erfolgreich, so daß der theoretische Kühlmediumbedarf nach wie vor bei ca. 30% des tatsächlichen Kühlmediumverbrauches liegt.

Die Aufgabe der Erfindung besteht darin, ein gattungsgemäßes Verfahren aufzuzeigen, das es ermöglicht, bei den einzelnen spezifischen Anwendungsbereichen mittels ge-

- 3 -                                          0225571

eigneter Vorrichtungen den Bedarf an Kühlmedium soweit zu
vermindern, daß er dem theoretischen Kühlmediumbedarf
sehr nahekommt.

Erfindungsgem. erfolgt die Lösung der Aufgabe dadurch,
daß ohne Verwendung eines Zwischenkühlmediums mit einer
dem kondensierenden Medium angepaßten Kühltemperatur als
Kühlmedium ein Stoff mit zur Kondensations-und/oder Verfestigungstemperatur ausreichend niedriger Verdampfungstemperatur verwendet wird, das am Austritt aus dem Wärmetauscher die Temperatur des Kondensatmediums besitzt und
zur Vermeidung einer Rückverdampfung des Kondensats die
kondensatführenden Leitungen derart umspült, daß auch bei
unter der Verfestigungstemperatur des zu kondensierenden
Gases liegender Kühlmitteltemperatur eine ungewollte
Vereisung des Kondensats verhindert wird. Zur Herstellung
von Halogenlampen werden die im Lampenkörper befindlichen
verflüssigten Edelgase solange gekühlt, bis die Lampenkörper verschweißt sind.

In einer Ausgestaltung der Erfindung wird das Kühlmedium
in dem Wärmetauscher durch ein Doppelrohr geführt, das
aus einem mit dem Kühlmediumeintrittsstutzen verbundenen
Kernrohr und einem mit den Kühlmediumaustrittsstutzen
verbundenen Mantelrohr besteht. Hierdurch wird ein Vereisen des Wärmetauschers verhindert. Zur dosierten Befüllung von Produkthohlkörpern kann am Flüssiggasausgang
des Wärmetauschers ein Dosierventil angeordnet werden.

Durch die erfindungsgemäße Lösung sind schnellere Taktzahlen und somit auch erhöhte Produktionsgeschwindigkeiten sowie die Lagerhaltung flüssiger Edelgase möglich.
Der Kühlmittelbedarf wird um den Faktor 2 erheblich
reduziert. Die Produktionsanlagen können einfacher gestaltet werden, wodurch sich geringere Investitionskosten
ergeben. Die Ausschußquote wird verringert da eine Versprödung des Materials von Produkthohlkörpern nicht mehr erfolgt. Ferner ist eine eindeutige reproduzierbare Mengendosierung von Edelgas oder Gasgemischen möglich. Von Vorteil ist auch die Möglichkeit der weiteren Ausnutzung des aus dem Wärmetauscher austretenden Kühlmediums bei anderen Verfahrensschritten.

Weitere Ausgestaltungen der Erfindung werden in den abhängigen Ansprüchen beschrieben und nachstehend anhand der Zeichnungen näher erläutert. Es zeigt

Fig. 1  die Vorrichtung zur Durchführung des erfindungsgemäßen Verfahrens in einem schematischen Anlagenschaltbild,

Fig. 2  die Ausbildung des Wärmetauscherrohrs für das Kühlmedium in einer schematischen Seitenansicht im Schnitt,

Fig. 3  den Temperaturverlauf an einem Wärmetauscherrohr nach Fig. 2,

Fig. 4  eine Ausbildung eines dem Wärmetauscher nachgeschalteten Dosierventils in einer Seitenansicht im Schnitt.

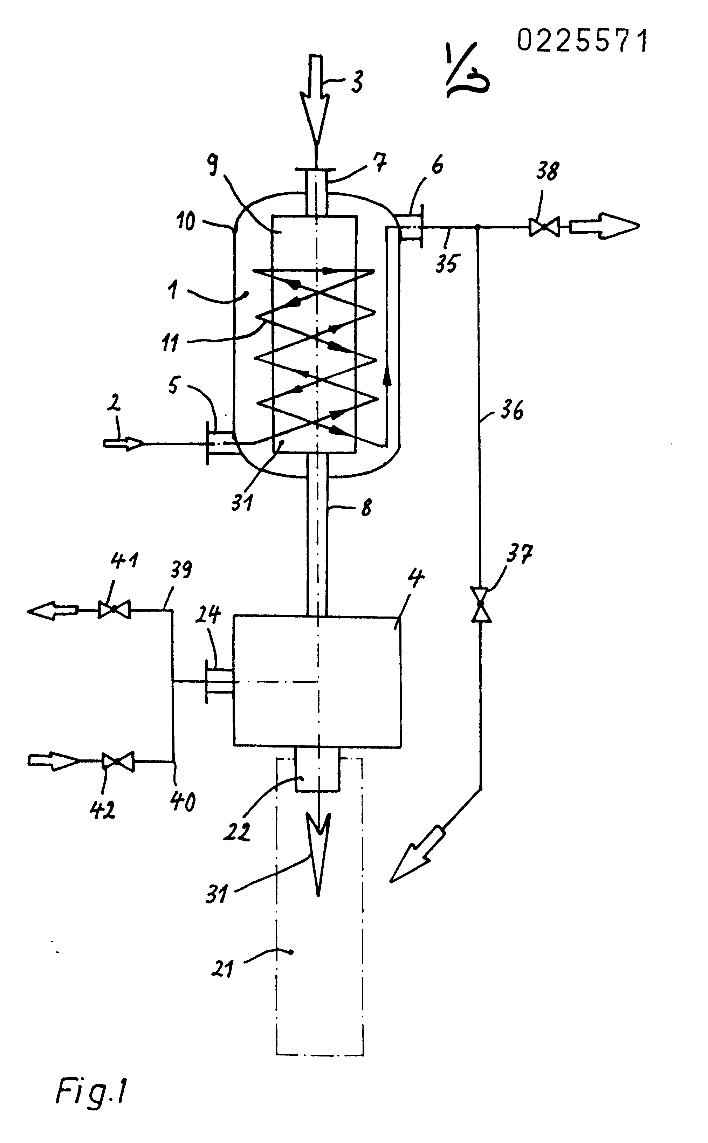

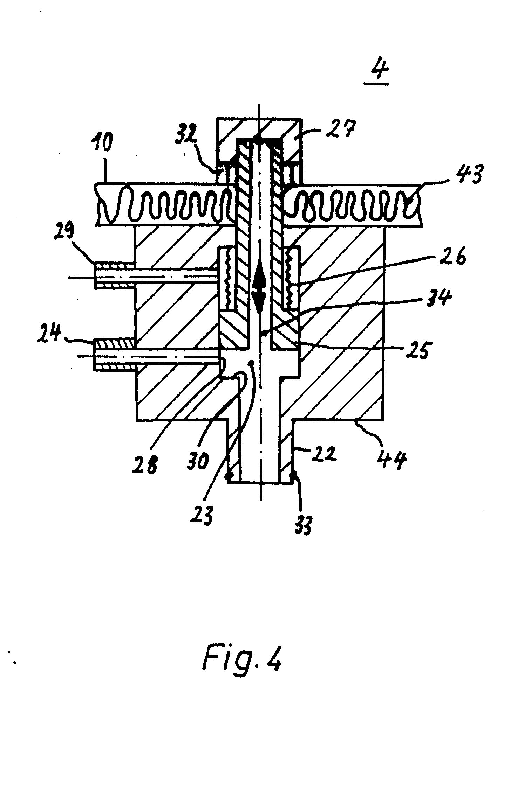

In Fig. 1 ist schematisch eine Vorrichtung dargestellt, mittels derer in der Gasphase befindliche kondensierbare Stoffe, insbesondere Edelgase und Kohlenwasserstoffgasgemische im Tieftemperaturbereich verflüssigt oder verfestigt werden können. Die Vorrichtung besteht aus einem Wärmetauscher 1 und einem Dosierventil 4. Der Wärmetauscher 1 weist einen Gaseintrittsstutzen 7 für ein Gas oder Gasgemisch 3 und einen Flüssiggasauslaßstutzen 8 sowie einen Kühlmediumeintrittsstutzen 5 und einen Kühlmediumaustrittsstutzen 6 auf. In dem Mantel 10 des Wärmetauschers 1, der zweckmäßigerweise mit einer Wärmedämmschicht 43 ummantelt ist (Fig. 4), ist eine Verflüssigungskammer 9 ausgebildet, die von einer Rohrschlange
11 umgeben ist. Die Verflüssigungskammer 9 ist mit dem
Gaseintrittsstutzen 7 und dem Flüssiggasauslaßstutzen 8,
die Rohrschlange 11 mit dem Kühlmediumeintrittsstutzen 5
und Kühlmediumaustrittsstutzen 6 verbunden.

Der Flüssiggasauslaßstutzen 8 ist mit einem Dosierventil
4 verbunden. An dessen Ventilausgang 22 ist ein Produkthohlkörper 21 angedeutet, der z. B. ein Lampenkörper sein
kann, und mit dem aus der Verflüssigungskammer 9 austretenden Flüssiggas 31 befüllbar ist. An dem Dosierventil 4 ist ferner eine Anschlußleitung 24 vorgesehen,
an die eine Leitung 39 für einen Vakuumanschluß sowie
eine Leitung 40 für ein Spülmittel angeschlossen ist. Die
Leitungen 39, 40 sind durch Ventile 41, 42 absperrbar.

Die an den Kühlmediumaustritt angeschlossene Leitung 35
ist mittels eines Ventils 38 absperrbar. Zwischen dem
Ventil 38 und dem Kühlmediumaustrittstutzen 6 ist eine
Leitung 36 an die Leitung 35 angeschlossen, die ebenfalls
ein Ventil 37 aufweist und bis in den Bereich des Produkthohlkörpers 21 geführt ist. Das Kühlmedium 2, das in
den Kühlmediumseintrittstutzen 5 flüssig eingeführt wird,
tritt aus dem Kühlmediumaustrittsstutzen 6 gasförmig aus.

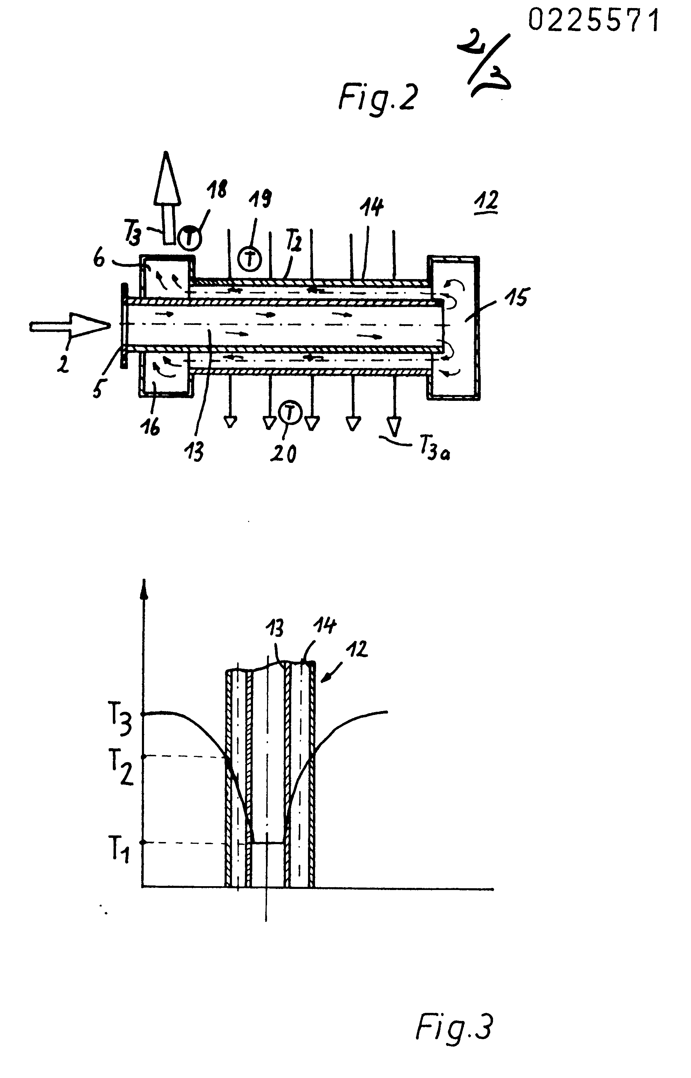

Um Edelgase oder Gasgemische zu verflüssigen, müssen sehr
exakte Kondensationstemperaturen erzeugt werden. In den
meisten Fällen ist die Temperatur des Kühlmediums 2
unter der Festpunkttemperatur der Edelgase oder aber der
Tripelpunkt liegt ungünstig zur Temperatur des Kühlmediums 2. Dieses führt in der Regel zu einer ungewollten
Verfestigung der Edelgase und somit zu einem Vereisen des
Wärmetauschers 1. Um dies zu verhindern, ist die Rohrschlange 11 aus einem Doppelrohr 12 gebildet, das aus einem mit dem Kühlmediumeintrittstutzen 5 verbundenen Kernrohr 13 und einem mit dem Kühlmediumaustrittstutzen 6 verbundenen Mantelrohr 14 besteht. Das Kernrohr 13 ist mit dem Mantelrohr 14 mittels einer Umlenkkammer 15 verbunden (Fig. 2). Der Kühlmediumaustrittstutzen 6 ist mit dem Mantelrohr 14 mittels der Umlenkkammer 16 verbunden. Mit Hilfe dieses Mantelrohrs 14 kann eine exakte Wandtemperatur $T_2$ an der Außenfläche 17 des Mantelrohrs 14 eingeregelt werden (Fig. 3). Diese Wandtemperatur $T_2$ entspricht der Temperatur des Kühlmediums 2 in der gasförmigen Phase und liegt ca. 1 bis 2°C unter der kritischen Verfestigungstemperatur. Damit werden unkontrollierte Kristallbildungen und daraus folgende Veränderungen der Wärmedurchgangskoeffizienten verhindert. Ein optimaler Wärmeübergang ist somit gewährleistet.

Darüberhinaus ist es aber auch möglich, eine Kristallisation bewußt herbei zu führen, in dem die Wandtemperatur $T_2$ unter den Festpunkt der Edelgase oder Gasgemische eingestellt wird. Je nach Kapazität des Wärmetauschers (Kilogramm Flüssigkeit/h) können verschiedene Wärmetauscherausführungen gewählt werden. Für kleinere Verflüssigungsleistungen eignet sich eine Spiralrohrausführung, für mittelgroße Verflüssigungsleistungen eine Plattentauscherausführung und für große Verflüssigungsleistungen eine Rohrbündelausführung. Für bewußte Kristallisationen können ergänzend konstruktive Hilfsmittel verwendet werden. Bei einer doppelten Aggregatausführung im wechselnden Betrieb kann der Vereisungsgrad über den Druckverlust ermittelt werden. Zum Reinigen vereister Wärmeaustauscherflächen können Kratzer oder Schaber dienen.

Zur optimalen Regelung der Temperatur im Wärmetauscher müssen die Temperaturen der den Wärmetauscher durchströmenden Mittel aufeinander abgestimmt werden, die

Temperatur $T_2$ zur Grenztemperaturbestimmung und die Temperatur $T_3$ zur Regulierung des Kühlmediumeintritts, wobei bei Erreichung der Solltemperatur $T_2$ die Kühlmediumzufuhr unterbrochen wird. Es kann auch die Temperatur $T_2$ zur Grenztemperaturbestimmung und die Temperatur $T_{3a}$ zur Regulierung des Kühlmediumeintritts verwendet werden. Wie bereits beschrieben, ist die Temperatur $T_2$ der Temperatur $T_{3a}$ übergeordnet.

Eine Ausführungsform eines Dosierventils 4 ist in Fig. 4 dargestellt. Dieses Dosierventil 4 ist als Kolbenventil ausgebildet, wobei der Kolben 25 ein Hohlkolben ist. Die Betätigung des Dosierventils 4 erfolgt pneumatisch über eine Steuerleitung 29. Die mit dem Ventilausgang 22 verbundene Kolbenkammer 23 ist mit der bereits erwähnten Anschlußleitung 24 für eine Vakuumpumpe und eine Spüleinrichtung verbunden. Der Kolben 25 ist mittels einer Zugfeder 26 zum Verschließen des Flüssiggasauslaßstutzens 8 auf ein Gegenlager 27 im Flüssiggasauslaßstutzen 8 preßbar. In dem Mantel des Gegenlagers 27 sind Durchbrechungen 32 ausgebildet, durch die bei auf den Kolbenkammernboden 30 gedrücktem Kolben 25 Flüssiggas strömt und durch die Durchbrechung 34 des Kolbens 25 zum Ventilausgang 22 gelangt. Der Ventilblock 44 ist direkt an dem Mantel 10 des Wärmetauschers 1 angesetzt. An dem Ventilausgang 22 ist eine Halteeinrichtung 33 ausgebildet, an der ein Produkthohlkörper 21 wie z. B. ein Lampenkörper zum definierten Befüllen mit Flüssiggas lösbar befestigt werden kann. Mittels dieses Dosierventils 4 ist es möglich, den jeweiligen Produkthohlkörper 21 zu evakuieren und anschließend mit reinem Gas wie z. B. Stickstoff zu spülen und dann nach vorgegebener Taktzeit mit verflüssigten Edelgasen oder Gasgemischen exakt dosiert zu befüllen. Diese drei Funktionen können auf kleinstem Raum realisiert werden. Die Dosierung ist abhängig vom Kolbenhub des Kolbens 25. Gasseitig muß darauf geachtet werden, daß im Vakuumzustand kein verflüssigtes Produkt in die gasführenden Leitungen dringt. Dieses wird durch die Verwendung des Kolbens 25 als Schließorgan verhindert. Es ist auch möglich, Dosierventile 4 zu verwenden, die andersartig ausgestaltet sind. So kann z. B. ein 5/2-Wege-Ventil mit metallisch dichtenden Oberflächen und pneumatischer getakteter Ansteuerung verwendet werden. Wärmetauscher 1 und Dosierventil 4 werden zweckmäßigerweise in vakuumisolierter Bauweise erstellt um eine mögliche Gasbildung in den flüssigkeitführenden Rohrleitungen zu verhindern.

Die Anwendung des beschriebenen Verfahrens und der Vorrichtung ist nicht auf das definierte Befüllen von Produkthohlkörpern 21 beschränkt. So ist es auch möglich, das aus dem Wärmetauscher 1 austretende Flüssiggas 31 mit im Wärmetauscher 1 eingestellter Temperatur zur Kühlung definierter Flächenabschnitte von Körpern beliebiger Art zu verwenden. Durch diese Eigenschaft kann das Verfahren und die Vorrichtung auch z. B. im Bereich der Rheumatherapie eingesetzt werden. Hier ist es bekannt, auf rheumatisch erkrankte Glieder kaltes Gas aufzusprühen, wobei im Regelfall Flüssigstickstoff mit einer Temperatur von ca. -180°C verwendet wird. Mittels der beschriebenen Vorrichtung ist es möglich, die Temperatur des aus dem Wärmetauscher 1 austretenden Flüssiggases 31 in einem Bereich von -60°C bis -188°C mit einer Toleranz von +/- 1°C einzustellen. Die therapeutische Behandlung kann somit individuell auf die jeweilige spezifische Ausbildung der Rheumaerkrankung durch entsprechende Dosierung der Temperatur des Flüssig-Stickstoffes abgestellt werden.

**PATENTANSPRÜCHE**

1. Verfahren zur Verflüssigung oder Verfestigung von bei Normaltemperatur in der Gasphase befindlichen kondensierbaren Stoffen insbesondere Edelgasen und Kohlenwasserstoffgasgemischen im Tieftemperaturbereich mittels eines Kühlmediums wie Stickstoff, Argon, $CO_2$ od. dgl., das den kondensierbaren Stoff in einem Wärmetauscher verflüssigt, der dann in einen Produkthohlkörper eingebracht wird wobei das aus dem Wärmetauscher austretende durch den Wärmeaustausch in der Gasphase befindliche Kühlmedium ganz oder teilweise auf die äußere Oberfläche des Produkthohlkörpers geleitet und der in diesem befindliche Stoff in der flüssigen Phase gehalten wird, dadurch gekennzeichnet, daß ohne Verwendung eines Zwischenkühlmediums mit einer dem kondensierenden Medium angepaßten Kühltemperatur als Kühlmedium ein Stoff mit zur Kondensations-und/oder Verfestigungstemperatur ausreichend niedriger Verdampfungstemperatur verwendet wird, das am Austritt aus dem Wärmetauscher die Temperatur des Kondensatmediums besitzt und zur Vermeidung einer Rückverdampfung des Kondensats die kondensatführenden Leitungen derart umspült, daß auch bei unter der Verfestigungstemperatur des zu kondensierenden Gases liegender Kühlmitteltemperatur eine ungewollte Vereisung des Kondensats verhindert wird.

2. Verfahren nach Anspruch 1, dadurch gekennzeichnet, daß das aus dem Wärmetauscher austretende in der Gasphase befindliche Kühlmedium für weitere Verfahrensschritte wie für die Lampenherstellung, die Kondensation hochwertiger Kohlenwasserstoffe zu deren Wiedergewinnung, die Kühlung chemischer Produkte, die Erzeugung exakter Kühlgastemperaturen in

0225571

der Blasformung oder Extrudertechnik, die Abluftreinigung bei Tanklagern mit kondensierbaren Verdampfungsprodukten, die Rückverflüssigung von Helium
in der Elektronenbeschleunigertechnik, die Erzeugung
von $CO_2$- Granulat hoher Dichte, die Inertisation und
Reinigung von Anlagenteilen od. dgl. verwendet wird.

3. Verfahren nach Anspruch 1 und 2, dadurch gekennzeichnet, daß durch eine getaktete Dosierung der kondensierbaren Stoffe wie Edelgase oder Gasgemische in den
Produkthohlkörpern eine reproduzierbare Füllmenge
eingebracht und damit ein reproduzierbarer Fülldruck
eingestellt wird.

4. Anwendung des Verfahrens nach Anspruch 1 bis 3 zur
Herstellung von Halogenlampen u. dgl., bei dem die
zur Befüllung der Lampenrohlinge erforderlichen
Edelgase durch das Kühlmedium verflüssigt und verflüssigt in die Lampenkörper eingebracht werden, die
mit dem aus dem Wärmetauscher austretenden in der
Gasphase befindlichen Kühlmedium  solange gekühlt
werden, bis die Lampenkörper verschweißt sind, dadurch gekennzeichnet, daß bei Eintritt einer Entmischung der Gasmischung, deren Flüssigtemperatur
unter dem Festpunkt der hochsiedenden Bestandteile
liegt, die hochsiedenden Gemischkomponenten gasförmig
und die niedrig siedenden Gemischkomponenten flüssig
in den Lampenkörper dosiert werden, wobei zur Einhaltung einer exakten Gemischdosierung die niedrig
siedenden Gemischkomponenten die höher siedenden
Komponenten im Lampenkörper verfestigen.

5. Anwendung des Verfahrens nach Anspruch 4, dadurch gekennzeichnet, daß zur Aufrechterhaltung einer genauen Gesamtmischung auch bei Verdampfungsverlusten die niedrig siedende Gemischkomponente etwas höher als erforderlich flüssig zudosiert wird.

6. Vorrichtung zur Durchführung des Verfahrens nach Anspruch 1 bis 5, dadurch gekennzeichnet, daß das Kühlmedium (2) in dem Wärmetauscher (1) in einem Doppelrohr (12) geführt ist, das aus einem mit dem Kühlmediumeintrittsstutzen (5) verbundenen Kernrohr (13) und einem mit dem Kühlmediumaustrittstutzen (6) verbundenen Mantelrohr (14) besteht.

7. Vorrichtung nach Anspruch 6, dadurch gekennzeichnet, daß das Kernrohr (13) mit dem Mantelrohr (14) mittels einer Umlenkkammer (15) verbunden ist.

8. Vorrichtung nach Anspruch 6 und 7, dadurch gekennzeichnet, daß das Mantelrohr (14) mit dem Kühlmediumaustrittstutzen (6) mittels einer Umlenkkammer (16) verbunden ist.

9. Vorrichtung nach Anspruch 6 bis 8, dadurch gekennzeichnet, daß zur Regelung der Menge des den Wärmetauscher (1) durchströmenden Kühlmediums (2) am Kühlmediumaustrittsstutzen (6) und der Außenfläche (17) des Mantelrohrs (14) und ggf. dem Flüssiggasauslaßstutzen (8) ein Temperatursensor (18, 19, 20) einer Regeleinrichtung angeordnet ist, wobei mittels des Temperatursensors (18) der Zufluß des Kühlmediums (2) zur Einstellung der Verflüssigungstemperatur des zu kondensierenden Mediums, mittels des Temperatursensors (19) der Zufluß des Kühlmediums (2) in Abhängigkeit von der Wandtemperatur des Mantelrohr (14) unter Berücksichtigung der Vereisungstemperatur des zu kondensierenden Mediums in Vorrangschaltung gegenüber dem Temperatursensor (18) und mittels des Temperatursensors (20) zur Niveauüberwachung im Verflüssiger die Temperatur des verflüssigten Mediums in Abhängigkeit von dem im Verflüssiger bestehenden Druck regelbar ist.

10. Vorrichtung nach Anspruch 6 bis 9, insbesondere zur Durchführung des Verfahrens nach Anspruch 1 bis 6 zum Befüllen von Produkthohlkörpern wie Lampenkörpern mit definierten Mengen verflüssigter Edelgase oder Gasgemische, dadurch gekennzeichnet, daß am Flüssiggasaustritts (8) des Wärmetauschers (1) ein Dosierventil (4) angeordnet ist.

11. Vorrichtung nach Anspruch 10, dadurch gekennzeichnet, daß das Dosierventil (4) als Kolbenventil ausgebildet ist.

12. Vorrichtung nach Anspruch 10 und 11, dadurch gekennzeichnet, daß das Dosierventil (4) pneumatisch, hydraulisch oder elektrisch betätigbar ist.

13. Vorrichtung nach Anspruch 10 bis 12, dadurch gekennzeichnet, daß die mit dem Ventilausgang (22) verbundene Kolbenkammer (23) mit mindestens einer Anschlußleitung (24) für eine Vakuumpumpe und eine Spüleinrichtung verbunden ist.

14. Vorrichtung nach Anspruch 10 bis 13, dadurch gekennzeichnet, daß der Kolben (25) als Hohlkolben ausgebildet und mittels einer Zugfeder (26) zum Verschließen des Flüssiggasauslaßstutzens (8) auf ein Gegenlager (27) im Flüssiggasauslaßstutzen (8) preßbar ist.

15. Vorrichtung nach Anspruch 14, dadurch gekennzeichnet, daß der Kolben (25) mittels Steuerluft oder einem Steuerfluid unter Verschließen der Öffnung (28) der Anschlußleitung (24) in der Kolbenkammer (23) gegen den Kolbenkammerboden (30) derart preßbar ist, daß Flüssiggas (31) durch am Gegenlager (27) ausgebildete Durchbrechungen (32) und die mittige Durchbrechung (34) des Kolbens (25) zum Ventilausgang (22) strömt.

16. Vorrichtung nach Anspruch 10 bis 15, dadurch gekennzeichnet, daß an dem Ventilausgang (22) eine Halteeinrichtung (33) für einen Produkthohlkörper (21) angeordnet ist.

17. Anwendung der Vorrichtung nach Anspruch 6 bis 14, dadurch gekennzeichnet, daß das aus dem Wärmetauscher (1) austretende Flüssiggas (31) mit im Wärmetauscher (1) eingestellter Temperatur zur Kühlung definierter Flächenabschnitte von Körpern beliebiger Art verwendet wird.

18. Anwendung der Vorrichtung nach Anspruch 17, dadurch gekennzeichnet, daß als Flüssiggas (31) Flüssig-Stickstoff verwendet wird, der zur Rheumatherapie auf rheumatisch erkrankte Glieder eines menschlichen Körpers geleitet wird.

Fig.1

*Fig.2*

*Fig.3*

Fig.4